# EUROPEAN PATENT APPLICATION

(11) **EP 3 970 507 A1**
(43) Date of publication of application: **23.03.2022**
(21) Application number: 20196596.9
(22) Date of filing: 17.09.2020
(51) Int. Cl.: A23L 5/30, A23L 33/105, A23L 33/11, A61K 8/18, A61K 8/97, A61K 36/00, A61K 36/02, A61K 36/06, A61K 36/09, A61K 36/63, A61P 17/16, A61P 17/18, A61Q 19/00, B01D 11/04

(54) **EXTRACTING PROCESS OF NATURAL COMPOUNDS ASSISTED BY INFRARED AND INFRASOUND**

(71) Applicant: HallStar Beauty and Personal Care Innovations Company, Chicago, IL 60606 (US)
(72) Inventor: CELHAY, Clément, 34000 MONTPELLIER (FR); ROSSIGNOL-CASTERA, Anne, Françoise, Marie, 34400 LUNEL (FR)
(74) Representative: Lavoix

(57) **Abstract**

The present invention relates to process of extraction of natural compounds from raw materials having a water content between 0 % and 20 %, said process comprising:
a) mixing and impregnating the raw materials by dispersing said raw materials with a liquid extracting solvent, under agitation, the ratio between the raw materials and the solvent being comprised between 1:1 and 1:20 in mass/mass,
wherein step a) is preceded by:
a0) activating by InfraRed the raw materials, said raw materials being in the form of a powder, at a temperature below their ignition point, with an agitation; and/or
wherein step a) is followed by:
b) extracting the natural compounds by submitting the mix obtained at step a) to excitation by InfraRed and/or sonic vibration by InfraSound, under an agitation.

## Description

The present invention relates to an extracting process of natural compounds contained in a raw material thanks to a liquid extracting solvent, without chemical transformation and with the intensification by a physical technique using InfraRed and InfraSound.

More precisely, the present invention relates to a process of extraction of natural compounds from raw materials having a water content between 0 % and 20 %, said process comprising:
a) mixing and impregnating the raw materials by dispersing said raw materials with a liquid extracting solvent, under agitation, the ratio between the raw materials and the solvent being comprised between 1:1 and 1:20 in mass/mass,
wherein step a) is preceded by:
a0) activating by InfraRed the raw materials, said raw materials being in the form of a powder, at a temperature below their ignition point, with an agitation; and/or
wherein step a) is followed by:
b) extracting the natural compounds by submitting the mix obtained at step a) to excitation by InfraRed and/or sonic vibration by InfraSound, under an agitation.

It further relates to a pharmaceutical, cosmetic, cosmeceutical, nutraceutical or food composition, comprising an extract enriched in natural compounds obtained by the process according to the invention.

Natural extracts are broadly used in pharmaceutics, cosmetics, cosmeceutics, nutraceutics, human nutrition and pet food industries. These domains are concerned by specific regulations about the toxicity of the extracts, the environmental impact of the processes and subjected to expectations about the bioactive substances' contents.

Traditional techniques exist for the recovery of actives from plants, such as maceration, heated solvent extraction, hydro-distillation, but the extraction times can be long, with low extraction yields. Moreover, most of these techniques need the use of controversial organic solvents to obtain natural substances. Several eco-friendly, efficient and economic extracting techniques of natural compounds have then been developed, using high temperatures and pressures, microwaves, ultrasounds, supercritical and subcritical conditions, enzymes, molecular distillation.

These techniques are complementary because they extract a selected fraction of natural molecules (polyphenols, flavonoids, tannins, carotenoids, xanthophylls, phytosterols, polar lipids, enzymes, minerals, salts, and more) according to the affinity of these compounds with the solvent used.

The European patent EP2413706B1 describes an extracting process assisted by microwaves and ultrasounds, using a natural oil or fat as solvent, with a prior step of cryogrinding of the plant material. This process, with scalable applications, provides oily extracts concentrated in phenolic and other bioactive compounds, with low oxidation of the oily solvent.

Electromagnetic (transversal) waves on the one hand, and sonic (longitudinal) waves, on the other hand, offer physical and non-chemical technologies usable for natural substances extraction. Consequently, they are safe and clean, and they quickly activate the part of the plant compounds that must be pushed outward, without affecting the quality of the solvent. Thus, extracting with the assistance of an energetic wave is a way to respect the principles of "Green Chemistry" in plant extraction, in that it must be non-chemical, non-hazardous, waste free, with low harmful emission, and more (Anastas, P.T.; Warner, J.C. Green Chemistry: Theory and Practice. Oxford University Press: New York, NY, USA, 1998.).

InfraRed is an electromagnetic wave characterized by a wavelength between 7.6 µm to 1000 µm. This energy form exposes surfaces which absorb this energy and that are heated by convection (Escobedo, R.; Miranda, R.; Martinez, J. InfraRed irradiation: toward green chemistry, a review. Int. J. Mol. Sci. 2016, 17, 4539.) and also activates different vibrational modes of molecules. (Cai, Y.; Yu, Y.; Duan, G.; Li, Y. Study on InfraRed-assisted extraction coupled with high performance liquid chromatography (HPLC) for determination of catechin, epicatechin, and procyanidin B2 in grape seeds. Food Chem. 2011, 127, 1872-1877).

Concerning the recovery of secondary metabolites, InfraRed rays, from near InfraRed to far InfraRed, have been tested for the solid-liquid extraction of polyphenolic compounds, flavonoids, cinnamic acids and sugars, from various vegetable species (Martinez, J.; Miranda, R. Infrared Irradiation, an excellent alternative green energy source. Green Chemistry Application - IntechOpen. DOI: http://dx.doi.org/10.5772/intechopen.83805) and notably from wastes, such as pomace (Cheaib, D.; EI Darra, N.; Rajha, H.N.; EI-Ghazzawi, I.; Mouneimne, Y.; Jammoul, A.; Maroun, R.G.; Louka, N. Study of the selectivity and bioactivity of polyphenols using InfraRed assisted extraction from apricot pomace compared to conventional methods. Antioxidants. 2018, 7, 174-186.) by several extracting solvents such as methanol, ethanol or water.

According to the selectivity of the extraction for specific compounds provided by the technique, it is possible to modulate the sample extracted towards higher amounts of targeted compounds and avoiding unwanted substances (Martinez, J.; Rosas, J.; Perez, J.; Saavedra, Z.; Carranza, V.; Alonso, P. Green approach to the extraction of major capsaicinoids from habanero pepper using near-InfraRed, microwave, ultrasound and Soxhlet methods, a comparative study. Nat Prod Res. 2019, 33(3), 447-452).

In addition to the good extraction yields, the economic interest of InfraRed Assisted Extraction (IRAE) is the immediate response time of the heat source (less than 1 second in some cases) and the longer life-time of emitters (Córdova, M.O.; Flores Ramírez, C.I.; Bejarano, B.V.; Arroyo Razo, G.A.; Perez Flores, F.J.; Tellez, V.C.; Ruvalcaba, R.M. Comparative study using different InfraRed zones of the solventless activation of organic reactions. Int J Mol Sci. 2011, 12(12); 8575-8580.). Finally, compared to traditional methods, this technique is affordable, easy, safe and allows to reduce reaction times, to optimize production yields and avoid undesirable byproducts.

InfraSound is a sonic wave of low frequency, below 20 Hz, i.e. 20 cycles of vibration per second, which is not audible by the human ear (Roosth, S. Nineteen Hertz and Below: An InfraSonic History of the Twentieth Century. Resilience: A Journal of the Environmental Humanities. 2018, 5(3), 109-124.). As a sonic wave, InfraSound transports energy through the air and the matter by vibrating and by successive compression-dilatation steps. This mechanical effect can be used for industrial applications. According to the patent US6063894A, InfraSound does not generate a cavitation effect, like UltraSound, but induce a softer vibration to the matter. A low frequency (900-1000 Hz) device emitting InfraSonic vibrations producing violent agitation of a liquid solution evidences the propagation of a considerable sonic stream. Patent US3491022A describes the effect of InfraSound on reverse osmosis between two chambers of salted water separated by a permeable membrane. The observed effect is the maximization of unclogging of the membrane, allowing more exchanges between the two chambers. The low frequency vibrational energy of InfraSound produces turbulence and friction at the interface between the chamber containing the concentrated saltwater and the membrane. InfraSound also brings the energy needed to force freshwater flow through a permeable membrane. These low frequency sound waves act on substances transfers thanks to percussions and rarefactions episodes. These phenomena occur without the rapid and severe destructive effects associated with sonic waves of higher frequencies.

Although the strict InfraSound band is below 16-20 Hz, low frequency vibrations from about 0 to 500 cycles per seconds, often 1000 Hz, are considered as InfraSound:
- Patent US3491022A claimed the use of low frequency vibrational energy in the range from about 1 to about 500 cycles per seconds as being InfraSound.
- Patents FR1131551 and FR1437460 refer to a machine using synchronized batteries of sonic emitters to focus the sound beam on any desired target and leading to investigations involving electronic speakers at a frequency of 256 cycles per seconds to generate InfraSonic conditions.
- The device used in patent US6063894A produced vibrations in the range of approximately 900-1000 Hz, regarded as InfraSound by the inventors.

To date, no recovery of natural compounds performed thanks to InfraSound Assisted Extraction (ISAE) has been reported.

The invention relates to a process of extraction of natural compounds from raw materials having a water content between 0 % and 20 %, said process comprising:
a) mixing and impregnating the raw materials by dispersing said raw materials with a liquid extracting solvent, under agitation, the ratio between the raw materials and the solvent being comprised between 1:1 and 1:20 in mass/mass,
wherein step a) is preceded by:
a0) activating by InfraRed the raw materials, said raw materials being in the form of a powder, at a temperature below their ignition point, with an agitation; and/or
wherein step a) is followed by:
b) extracting the natural compounds by submitting the mix obtained at step a) to excitation by InfraRed and/or sonic vibration by InfraSound, under an agitation.

It further relates to a pharmaceutical, cosmetic, cosmeceutical, nutraceutical or food composition, comprising an extract enriched in natural compounds obtained by the process according to the invention.

The present invention allows a selective recovery of well-preserved bioactive metabolites, with high yields, for pharmaceutical, cosmetical, cosmeceutical, nutraceutical, human and animal food applications. For the first time InfraRed is applied to a solvent, especially an oily solvent, InfraSound is applied to an extraction, and in particular, InfraSound can be used in synergy with InfraRed.

The invention will be further understood with reference to the drawings of preferential design, in which:
**Fig. 1** is a schematic diagram of a first apparatus in accordance with the present invention, with repetitions of Near and Medium InfraRed circular tubes or sheaths around the extracting tank, and an InfraSound emitting diving probe.
**Fig. 2** is a schematic diagram of a second version of the apparatus shown on Fig.1, but with Far InfraRed glass or ceramic elements, instead of the tubes.
**Fig. 3** is a schematic diagram of a third version of the apparatus shown on Fig.1, but with InfraSonic transducers placed at the bottom of the extracting tank, instead of the top.

The present invention is directed towards a novel extracting technique using one or two physical technologies, and the combination of both. These techniques are solely physical, and the use of chemical additive is not needed.

The present invention can be applied to a wide range of solvents, preferably oily solvents, preferentially green, natural or agro-sourced, such as vegetable oils and fats (including waxes and butters), fatty acids, fatty esters, fatty alcohols, glycerides and any other eco-solvent.

Also provided is the selectivity of the family of natural compounds extracted from the same matrix, according to the solvent used in combination with this extracting method.

This effect is due to the response of the solvent, on the one hand, and the natural extractable constituents, on the other hand, to the excitation promoted by the energy sources. This invention thus offers a wide range of complementary extracts, regarding the profile of the compounds extracted and the activities of the extracts.

Another advantage of the invention is that the substances thus obtained are highly concentrated and preserved. The technologies used lead to no or low degradation of the extracted substances and of the solvent. The present invention allows short treatment times that limit the degradation of the extracted compounds and of the extracting solvent. In the same time, the invention limits the intensity of odor and color of the obtained extract.

Finally, because of short extraction times, high yields and highly reactive energy sources (low response time), the invention allows low consumption of material and energy, that leads to an economic and energy-saving extracting process.

As already mentioned, the object of the invention is a process of extraction of natural compounds from raw materials having a water content between 0 % and 20 %, said process comprising:
a) mixing and impregnating the raw materials by dispersing said raw materials with a liquid extracting solvent, under agitation, the ratio between the raw materials and the solvent being comprised between 1:1 and 1:20 in mass/mass,
wherein step a) is preceded by:
a0) activating by InfraRed the raw materials, said raw materials being in the form of a powder, at a temperature below their ignition point, with an agitation; and/or
wherein step a) is followed by:
b) extracting the natural compounds by submitting the mix obtained at step a) to excitation by InfraRed and/or sonic vibration by InfraSound under an agitation.

By "raw materials" is meant any solid containing extractable compounds of interest. The raw material can be constituted by one matrix or a blend of matrices, according to the molecular profile and the bioactivity desired for the final extract. Is preferred a green material, of natural origin, renewable, non-pollutant. Is also preferred a non-dangerous material, presenting no hazard such as inflammability, toxicity, carcinogenic-mutagenic-reprotoxic properties, etc.

The raw material can be all or a part of the following or a combination of them: a plant (leave, flower, bark, root, fruit, seed, tubercles, flour, press cake), a mineral (rock, sand, semi-precious or precious stone, semi-precious or precious metals), a fungus (lichen, other), an alga (microscopic and macroscopic ones), a bacterium or of animal origin (such as hive products, silk, milk, wool, horn, etc). The raw materials can also be an industrial byproduct (press cake from oil production, pomace from juice or wine industries, wastes from essential oil hydro-distillation), if it has not been subjected to any chemical treatment or any transformation modifying its chemical composition. The raw material of animal origin must be recovered in the respect of the animal's health and the integrity of its' living environment.

As a consequence, the invention relates to a process as described herein, wherein said raw materials are all or a part of a plant, a mineral, a fungus, an alga, a bacterium or of animal origin, an industrial byproduct that has not been subjected to chemical treatment.

In one embodiment, the raw material, when harvested humid, i.e. with a water content above 15% in mass, has previously been dehydrated for its stabilization to avoid degradation due to enzymatic or microbial activities. The process is advantageously realized on raw materials having a water content between 0 and 20 %, in particular between 3 and 18 % and more particularly between 5 and 15 %, in mass. The water content can be of natural origin, meaning the native water still contained in the material cells or structure after the drying step, or of exogen origin, by addition to the material, to ensure the right humidity level.

By "dispersing" is meant that the raw materials is in a dissociated form, and for example, that the raw materials is in a particulate, and, more particularly, in powder form.

In one embodiment, the raw material is dried and comminuted to a fine and regular powder to favorize its homogenous dispersion inside the solvent. This operation is led before step a) and before step a0), when step a0) is conducted.

By "liquid extracting solvent" is meant any substance allowing the dispersion and the solvation of the particles of raw material in it. The extracting solvent can be a liquid oily solvents allowing the solvation of the extractable compounds of interest from a natural matrix. The solvent can be constituted of one solvent or a blend of solvents, according to the fluidity and the affinity and solvency properties required for the extraction of the compounds of interest. Is preferred a green oily or fatty solvent, of natural origin, renewable, non-pollutant, presenting no hazard such as inflammability, toxicity, carcinogenic-mutagenic-reprotoxic properties, etc.

Even if the solvent is in a solid state at room temperature and atmospheric pressure, it can be liquefied before or during the application of the process. The temperature elevation occurring during the process allows and maintains the liquid state of the solvent. The solvent is preferably a green, In particular, all type of oily solvents can be used in the method according to the invention, in the respect of the safety of use, including inflammability and flashpoint, natural or agro-sourced solvent respecting the principles of the "Green Chemistry", and not an organic solvent with toxicity and safety risks.

In one embodiment of the invention, the profiles of compounds extracted is tunable according to the polarity and the solvency of the extracting liquid. These properties can be modulated by the blending of different solvents and the ratio of each one.

More particularly, solvent according to the process of the invention are chosen from oily solvents, such as vegetable liquid oil, vegetable butter, vegetable fat, natural wax, fatty acid, fatty ester, fatty alcohol, glyceride, any other fatty acid derivative or a combination of them.

By "natural compound" is meant any substance obtained from a natural raw materials. The compounds extracted can be non-polar, amphiphile or more polar molecules, or a mix of these substances that could have a synergistic activity, notably all secondary metabolites of plants like phenolic compounds (monophenols, polyphenols, flavonoids, phenolic acids, catechin, flavones, flavonol - in aglycone or glycosylated form), diterpenes, vitamins (A, E, C - in free or ester form), tannins, waxes, fatty acids, essential oils, organic acids, hydrophobic proteins, pigments (carotenoids, xanthophyll, lutein, zeaxanthin, chlorophyll), unsaponifiable compounds (free or esterified phytosterols, fatty alcohols, triterpenes, squalene), polar lipids (phospholipids, glycolipids, sphingolipids), enzymes and co-enzymes, oligo-elements, minerals, organic salts or a mix of these compounds.

In the context of the process according to the invention, the ratio between the raw material and the solvent in the starting mix of step a) is comprised between 1:1 and 1:20, preferably between 1:2 and 1:10, and preferentially between 1:3 and 1:5, expressed in mass/mass.

The "agitation" as mentioned in the context of the process according to the invention, can be performed with a mixing arm such as a blade or an anchor by the top of the reactor or a rotative stirrer at the bottom of the extracting tank. The process is performed at an agitation speed allowing the sufficient dispersion of the powder of raw material inside the solvent into a homogenous blend, to enhance the contact surface between the particles of raw materials and the solvent, but also to maximize the exposure of the raw materials particles to the energy forms. The extraction is preferably carried out using a rotation speed between 20 and 600 rpm.

The extracting step b) can be conducted under an oxygen-free or almost oxygen-free atmosphere. This means that it can be carried out in the presence of an inert gas or under vacuum or partial vacuum. The oxygen content must be low enough to avoid oxidation and combustion due to the temperature reached during the thermal treatment, and the light exposure. This implies the limitation of the risk of thermo-oxidation and photooxidation. The atmosphere inert can be obtained by a continuous nitrogen sweep, allowing the removal of present, or possibly formed oxygen. A closed reactor can be used, with a continuous extraction of oxygen by nitrogen flow in the head space or immersive stripping. The process can also be performed under vacuum. Proceeding as such confers an additional advantage, which is the washing out of the volatile compounds with a deodorization effect of the blend.

In one embodiment, step b) can be repeated more than once.

By "more than once" is meant in particular between 1 and 4, for example two times, three times or four times.

In particular, when step b) is repeated more than once, a passive diffusion step is performed between them at room temperature or with the cooling of the extracting tank. Cooling the extracting tank is preferred for the passive diffusion step.

By "passive diffusion step" is meant a maceration without energy application, except agitation.

By "room temperature" is meant a temperature comprised between 18°C and 25°C.

Step a0) is preferably performed at a temperature below the ignition, said ignition corresponding to the temperature at which the material starts combustion, in particular at a temperature between 20°C and 90°C in the limit of the alteration of the material in terms of color, odor and extractives content.

The duration of this step can be the time needed to reach the targeted temperature and can be extended with a temperature plateau. A short duration is particularly preferred, to promote uniform temperature elevation overall the material for an activation without any degradation, notably under 30 minutes, preferably under 15 minutes and more preferentially under 5 minutes. In one embodiment, step a0) is performed for a duration of between 5 and 30 minutes.

The duration is considered as short if we compare with the duration of a classical passive diffusion step that is several days or weeks.

Step a) is preferably carried out at room temperature.

Step b) is preferably carried out at a temperature between 20°C and 160°C, more preferably between 50°C and 140°C, and even more preferentially between 80°C and 120°C.

The duration of this step is the one allowing to reach the temperature needed and optionally maintained on a plateau, for the optimal extraction and preservation of the bioactive compounds of interest, in the limits of non-alteration of the solvent. A short duration is particularly preferred, notably under 90 minutes, preferably under 60 minutes and even more preferentially under 30 minutes.

In one embodiment, step b) is performed for a duration of between 5 and 90 minutes.

The passive diffusion is performed at room or lower temperature, to let the natural diffusion occur, and to allow a higher temperature elevation for the following step b), by lowering the initial temperature. The temperature will be even more diminished if the extracting blend is cooled by the use of a chillable double envelope. The diffusion step is preferably carried out between 5°C and room temperature, more preferably between 10°C and 20°C and even more preferentially between 12°C and 17°C - according to the melting point of the solvent.

Enough time for this phenomenon to occur is particularly preferred, notably above 30 minutes between each step b), preferably above 45 minutes and even more preferentially above 60 minutes.

"Energy form" and "energy source", will be used in the context of the process of the invention and correspond to a physical phenomenon allowing a mechanic, sonic and/or an electro-magnetic effect that promotes the activation of the raw materials or the extraction of solutes, natural compounds from the raw material towards the solvent, according the three modes of action that are heating, molecular excitation plus ionic conduction and vibration, as described below:
i) The heating, in particular by convection at a relatively high temperature enhances the solubilization power of the solvent and helps the contact between the solvent and the particles of raw materials, as well as the diffusion of the solutes towards the solvent.
ii) The excitation of reactive compounds (such as substances of interest in the raw materials or molecules of the solvent) also allows the breakage of the raw material's structure, the diffusion of solutes through the matrix and the penetration of the solvent inside the same matrix.
iii) The vibration allows the alteration of the structure of the raw materials for a better release of extractives, penetration of the solvent as well as the interaction of the solvent and the solutes.
iv) The excitation and the vibration also contribute to the temperature elevation, by the diffusion of the thermal energy from agitation or friction of molecules and compression-dilatation episodes of the solid material.

Finally, the energy also allows the durable micro-dispersion of the solutes in suspension inside the solvent.

By "micro-dispersion", is meant a homogenous blend of extracted compounds in suspension inside a solvent. The particle size is such that there is no or low decantation during time.

The use of innovative or original technologies in a closed reactor allows to reach, in a short time, the targeted temperature, a molecular excitation of reactive compounds and a sufficient vibration through the materials, and thus, to extract bioactive substances while limiting the phenomenon of thermic degradation, optionally under an optional oxygen-free or almost oxygen-free atmosphere.

Energy forms according to the process of the invention cover InfraRed and InfraSound.

InfraRed is the first original energy form, leading to said InfraRed Assisted Extraction (IRAE). InfraRed is produced by a different device according to the band of the wavelength. Near InfraRed or NIR (0.76 µm - 2 µm) can be generated by a tungsten, carbon or iron alloy filament in a quartz tube filled with halogen gas. Medium InfraRed or MIR (2 µm - 4 µm) can be issued from a chromium alloy filament inside a metal or quartz sheath. Far InfraRed or FIR (4 µm - 1000 µm) can be emitted by a metallic alloy resistance embedded in a hardened glass or a vitrified ceramic element. These technologies allow the temperature elevation by convection of heat produced by the rays emitted from the source, but also, as an electromagnetic wave, excites the vibrational state of reactive compounds on several dimensions.

In one embodiment, step b) comprises extracting the natural compounds by submitting the mix obtained at step a) to excitation by InfraRed together with heating by InfraRed convection between 20°C and 160°C.

Accordingly, the source of InfraRed according to the process of the invention can be chosen from a filament made of tungsten, carbon or iron alloys, embedded in a quartz tube filled with halogen gas, a chromium alloy filament inside a metal or quartz sheath, or a metallic alloy resistance embedded in a hardened glass or a ceramic element (plate or bulb).

The use of InfraRed can be at a power between 50 to 2000 W by kilograms of mix, and/or at a wavelength from NIR to FIR, i.e. 0.76 µm to 1000 µm.

InfraSound is the second original energy form, leading to said InfraSound Assisted Extraction (ISAE). The InfraSound source can be transducers, converting electrical input into sound signal, by moving and transmitting vibration.

In particular, in the process according to the invention, the source of InfraSound are transducers chosen from piezoelectric and inverse piezoelectric effect devices, magnetostrictive devices, sliding magnetic/moving coils or electrostatic devices.

These technologies allow the energy transfer by vibration inside the blend and thus, a subtle mechanical turbulence. The succession of compression and dilatation phases forces the penetration of solvent inside the matrix and the expulsion of solutes outside of the matrix.

The use of InfraSound can be at a power between 10 to 1000 W by kilograms of mix. The frequency can be between 0.1 Hz and 1000 Hz, preferably between 20 Hz and 500 Hz and preferentially from 50 Hz to 100 Hz.

As source of energy, InfraRed and InfraSound can be hazardous for human health in case of a direct exposure for a long time without protection. That is why, the use of these technologies is recommended along with the use of protective glasses for the eyes, hearing protection and safe exposure levels, under 70 dB.

In the process according to the invention, when performed together, InfraRed and InfraSound are performed sequentially or simultaneously.

InfraRed and InfraSound can be used together as defined in the process according to the invention and provide a synergistic effect on the recovery yields of extractives and/or time treatment reduction.

The use of InfraRed and InfraSound together as energy sources with synergistic effect lead to said InfraRed and InfraSound Assisted Extraction (IRISAE).

According to an advantage of the invention, these two technologies offer an antimicrobial effect by allowing the reduction of the contaminants that can be brought by the raw materials at the beginning of the extraction. The microbial content of the substances can be dramatically reduced in the extracts obtained, to guarantee a low level of contamination in time, during storage of the extract or the final formulation.

Accordingly, the process according to the invention can cover various embodiments, as illustrated in embodiments to i) to vi) below.

Embodiment i): the process comprises step a) and step b), wherein in step b), the mix obtained at step a) is submitted to excitation by InfraRed.

Embodiment ii): the process comprises step a) and step b), wherein in step b), the mix obtained at step a) is submitted to sonic vibration by InfraSound.

Embodiment iii): the process comprises step a) and step b), wherein in step b), the mix obtained at step a) is submitted to excitation by InfraRed and sonic vibration by InfraSound.

When performed together, InfraRed and InfraSound are performed sequentially or simultaneously.

As a consequence, in embodiment iii), the mix obtained at step a) can be submitted to sonic vibration by InfraSound first and then to excitation by Infrared (embodiment iii-1), or the mix obtained at step a) can be submitted to excitation by InfraRed first and then to sonic vibration by InfraSound (embodiment iii-b). In embodiment iii-c), they are performed simultaneously.

Embodiment iii-1-a: a passive diffusion step is performed between the sonic vibration by InfraSound and the excitation by InfraRed, at room temperature or with the cooling of the extracting tank.

Embodiment iii-2-a: a passive diffusion step is performed between the excitation by InfraRed and the sonic vibration by InfraSound, at room temperature or with the cooling of the extracting tank.

Embodiment iv: the process comprises step a) and step b), wherein in step b), the mix obtained at step a) is submitted to excitation by InfraRed, and wherein step b) is repeated twice.

Embodiment iv-a: a passive diffusion step is performed between the two excitations by InfraRed, at room temperature or with the cooling of the extracting tank.

Embodiment v: the process comprises step a) and step b), wherein in step b), the mix obtained at step a) is submitted to sonic vibration by InfraSound, and wherein step b) is repeated twice.

Embodiment v-a: a passive diffusion step is performed between the two sonic vibrations by InfraSound, at room temperature or with the cooling of the extracting tank.

Embodiment vi: the process comprises step a) and step b), wherein in step b), the mix obtained at step a) is submitted to excitation by InfraRed and sonic vibration by InfraSound, and wherein step b) is repeated twice.

Embodiment vi-a: a passive diffusion step is performed between the two steps b), at room temperature or with the cooling of the extracting tank.

All the possibilities involving the stoppage of one of the energy forms while the other is maintained, during step b), are included in the process of the present invention.

For example, the extraction step can use InfraRed for 20 minutes and InfraSound for 15 minutes. After 15 minutes, the InfraSound is stopped while the InfraRed is maintained during the last remaining 5 minutes. These examples are illustrative and do not represent the full scope of the method according to the invention.

In particular, embodiments i) to vi) can all be preceded by step a0).

At the end, the process can contain one or several steps of particles removal, stabilization and microbial neutralization. If the extract is conserved as a dry extract, these steps will be spin-drying, filtration, centrifugation, thermal treatment - or a combination of these techniques.

Without being bond by any theory but with an illustrative purpose only, below is a short description of the six mechanisms involved in the process according to the invention:
- The activation of the powder of raw material, by heating and exciting the constituents of the matrix.
- The active dispersion of the powder inside the extracting solvent obtained by a vigorous agitation.
- The vibration of the mix of powder and solvent to perform sequences of percussion and rarefaction, to unsettle the structure of the raw materials, and enhance the solvent's penetration and the mass transfer needed to push the solutes/nautral compounds outward the raw material.
- The elevation of temperature needed for the mass transfer and the diffusion of the solutes towards the solvent. Heating the extraction mix also allows the rupture of the raw material's structure, leading the diffusion of the solutes towards the solvent.
- The excitation of the molecules of the solutes to activate mass transfer, structure disruption and then, diffusion inside the solvent.
- The organization and stabilization of the extracted compounds inside the solvent.

The process, according to the invention, allows to obtain extracts, preferably oily extracts that are a homogenous micro-dispersion of active substances inside a solvent, preferably an oily or fatty solvent, stable against microbial development and oxidation. Antioxidants can also be added to the extracts, to avoid the oxidation of the solvent, kept as an active part of the final ingredient. Extracts can be conserved thanks to tocopherols or rosemary extracts or any other natural antioxidant system.

Natural raw materials, such as plant, as they grew in a non-controlled outside environment, may bring a high amount of undesirable microbial contaminants to the extraction mix. However, extracts for cosmetic, pharmaceutic, human and animal food products are required to present a very limited quantity of germs. As already mentioned above, another advantage of the present invention is the elimination of a large part of microbial contaminants by InfraRed and InfraSound, because of their physical and mechanical disruptive effect on cells. In addition to their actions of activation and extraction, these energy forms contribute to the microbial quality of the extracts, by an anti-microbial effect.

Another object of the invention is the use of the extracts of natural origin and/or organic certifiable, obtained according to the process of the invention, in formulas for alimentary, nutritional, nutraceutical, cosmetically, cosmeceutical, pharmaceutical and pet food applications.

The invention thus also concerns a pharmaceutical, cosmetic, cosmeceutical, nutraceutical or food composition, comprising an extract enriched in natural compounds obtained by the process according to the invention. Anhydrous or oily type applications such as serums, make-ups, creams, soaps, milks, lotions, shampoos are particularly contemplated.

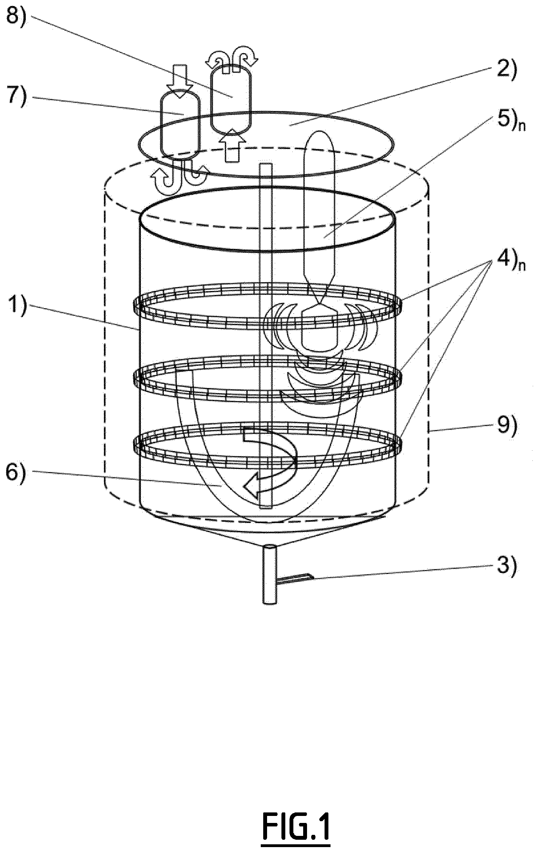

Examples of apparatuses that can be used in the process according to the invention will now be described with reference to **Figs. 1****,** **2** **and** **3****.** The primary component of the apparatus shown in **Fig.1** is a closed extracting vessel or tank **1),** that can be filled by the top, then protected by a mobile lid **2)** and emptied by a bottom valve **3).** The vessel is preferably made of high temperature- and high pressure-resisting glass, with a possible double envelope containing water, that can be cooled thanks to a chiller.

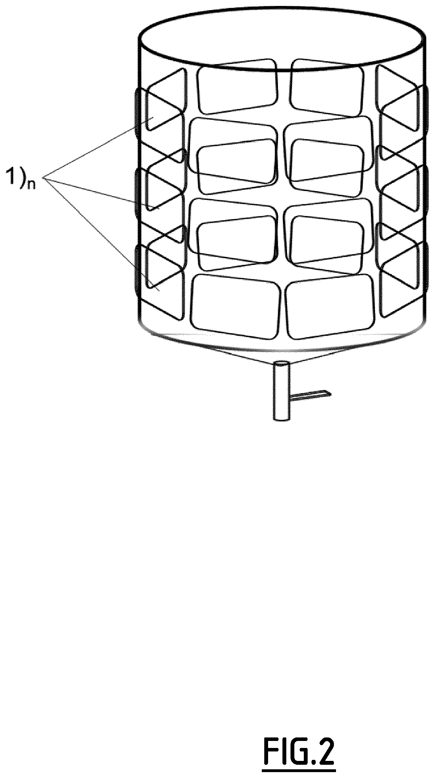

InfraRed emitters can be a stacking of circular tungsten/carbon/iron-halogen quartz tubes (NIR) or chromium filament in sheaths (MIR), as shown on **Fig. 1 - 4****)** or a circular disposition of metallic resistance in glass or ceramic elements, such as plates or bulbs (FIR), according to **Fig. 2 - 1****).** As the vessel or the tank is made of glass transparent to light, InfraRed rays emitted by the source are directly transmitted to the extraction mix. All the elements presented on **Fig.1** have not been reproduced on **Fig. 2****.** Only the detail that changes from one another, i.e. the InfraRed energy source, has been represented. Thus, **Fig. 2** must be read as a variant of **Fig. 1** with the same configuration, except the quartz tubes or sheaths that are replaced by the ceramic or glass elements. The number of three crowns of tubes or elements has arbitrarily been chosen for a better understanding of the drawings. However, it is possible to add or remove as much levels as needed, ***n*** being the number of repetitions.

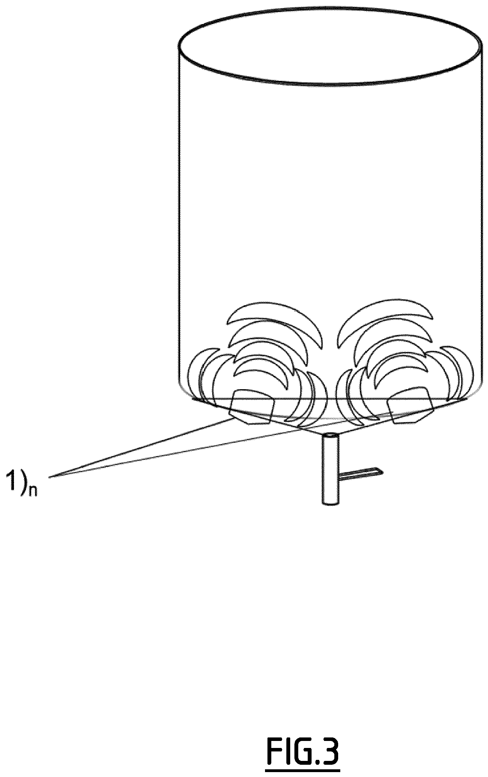

InfraSound can be produced by a source placed at the top of the tank **Fig. 1 - 5****),** with transmission of the sonic wave to the extracting mix by a diving probe, or a source placed at the bottom of the tank, on the external face **Fig. 3 - 1****),** to diffuse the vibrational energy by the wall of the tank. All the elements presented on **Fig. 1** have not been reproduced on **Fig. 3****.** Only the detail that changes from one another, i.e. the InfraSound energy source, has been represented. Thus, **Fig. 3** must be read as a variant of **Fig. 1** with the same configuration, except the place of the InfraSonic transducers. The number of transducers on the figures has arbitrarily been chosen for a better understanding of the drawings.

However, it is possible to add or remove as much transducers as needed, ***n*** being the number of repetitions.

The apparatus that can be designed according to the invention can correspond to any combination of the versions presented on **Fig.1****,** **2** and **3****,** such as:
- InfraRed quartz tube(s) or sheath(s) with InfraSound diving probe(s);
- InfraRed ceramic or glass element(s) with InfraSound diving probe(s) ;
- InfraRed quartz tube(s) or sheath(s) with InfraSound bottom-stucked transducer(s);
- InfraRed ceramic or glass element(s) with InfraSound bottom-stucked transducer(s).

Moreover, sources of NIR, MIR or FIR ca be used together. InfraSonic diving probes can also be used together with bottom-stucked transducers.

These are suggestions that do not exclude any other type of emitter or design made by a person skilled in the art, allowing to perform the process according to the present invention.

In the case of an addition of a double envelop, with quartz tubes or sheaths **Fig. 1 - 4****)** or the ceramic or glass elements **Fig. 2 - 1**) embedded inside the double envelop, it is preferable to cool the tank without activating the InfraRed source, that will produce heat. Cooling the extracting tank is preferred for the passive diffusion step .

Agitating system, such as anchors, helicoidal pales, screws or any other performant stirrers known by a person skilled in the art, is used to enhance the contact between the raw materials and the extracting solvent, on the one hand, and the exposure of the highest number of particles to the InfraRed and InfraSound waves. The stirrer **6)** is actioned by a motor at the top of the lid **2).** The headspace of the tank can be inerted with nitrogen injected by a pipe **7)** at the top of the lid and the gas containing oxygen, vapors and volatile compounds can be extracted by another pipe **8),** opened to the atmosphere.

As volatile compounds are not desired extractives and are non-dangerous, they can be expulsed to the ambient air. The whole tank can be covered by a visual and auditory protection envelope **9),** to prevent the operator from any risk involved by the use of InfraRed or InfraSound and to protect the extraction mix from the UV light.

The invention will be further illustrated by the examples below.

### EXAMPLES

Several acknowledged and normalized analytical procedures can be used to evaluate the quality of the obtained extracts. The extractive content can be determined with different methods according to the nature of these compounds. For instance, the total phenolic compounds content can be determined by the Folin-Ciocalteu dosage, the chlorophyll, xantophyll and carotenoids contents by UV-Visible absorption, and any specific molecule content by HPLC or GC-MS. The level of oxidation can be determined with the measurement of the peroxide value that evaluates the concentration of hydroxyperoxydes of fatty acids, according to NF ISO 3960. Concerning the microbial quality of the extracts, mesophilic aerobic counts can be determined according to NF EN ISO 21149 and NF ISO 16212, and the detection of the following contaminants can be performed by the respective subsequent methods : Coagulase positive *staphylococci* (NF EN ISO 22718), *Pseudomonas aeruginosa* (NF EN ISO 22717), *Candida albicans* (NF EN ISO 18416), *Escherichia coli* (NF EN ISO 21150).

### Example 1

100 g of olive leaves with a water content of 20% (m/m), cut with a hammer mill with a sieve of 800 µm, were mixed with 400 g of deodorized sunflower oil, at a ratio 1:4.

The mix was poured in a closed system (glass Erlenmeyer flask) surrounded by a circular quartz tube containing a filament and filled with halogen.

Step b) was carried out during 17 minutes, under Near InfraRed with a power of 1000 W, with a mechanical agitation of 200 rpm. The temperature went from 20°C to 161°C.

The liquid phase was separated from the exhausted plant material by filtration on a cellulosic paper of 1 µm porosity.

The total phenolic content went from less than 10 ppm (mg oleuropein equivalent/kg) for sunflower oil to 566 ppm (mg oleuropein equivalent/kg) for the oily extract obtained with the process.

The peroxide value of the sunflower oil before the extraction was of 6.0 +/- 0.5 meqO₂/kg and the one of the oily extract obtained with the process was of 7.7 +/- 0.5 meqO₂/kg, without any nitrogen sweep during the process.

The antiradical activity of the oily extract was 1652 µmoles Trolox equivalent/kg.

A microbial analysis of the extract was performed according to the respective norms for each contaminant, showed results below 100 units forming colony per gram in mesophilic aerobic counts, and the absence of Coagulase positive *staphylococci, Pseudomonas aeruginosa, Candida albicans* and *Escherichia coli.* A simple maceration performed without the application of the process led the presence of 20 (UFC/g) of mesophilic aerobic bacteria and mesophilic aerobic micro-organisms.

The same process has been performed on olive leaves with a water content of 7%, without addition of exogen water. The phenol content of extracts at temperature 80°C, 120°C and 160°C, respectively were: 49, 115, 737 ppm (mg oleuropein equivalent/kg).

The same process has been performed on 25 g of olive leaves in 500 g of sunflower oil, at a ratio 1:20. After 15 min of step b), the temperature went from 23°C to 122°C. The total phenols content was 56 ppm (mg oleuropein equivalent/kg).

### Example 2

125 g of powder of olive leaves, with a water content of 20% (m/m), were mixed with 500 g of deodorized sunflower oil, at a ratio 1:4.

Step b) was carried out during 15 minutes, under Near InfraRed with a power of 1000 W, with a mechanical agitation of 200 rpm. The temperature went from 21°C to 122°C.

A passive diffusion was then carried out for 70 minutes, with cooling of the flask by external contact with water at 15°C, during the last 15 minutes.

A second step b) was then performed during 15 minutes, under Near InfraRed. The temperature went from 32°C to 148°C.

The total phenolic content was 190 ppm (mg oleuropein equivalent/kg) after 5 minutes, 236 ppm (mg oleuropein equivalent/kg) after 10 minutes and 431 ppm (mg oleuropein equivalent/kg) after 15 minutes of exposure on the second step b), for the oily extract obtained with the process.

The antiradical activity of the oily extract was 1114 µmoles Trolox equivalent/kg.

The microbial activity of the extract was compliant with the norms.

An oily extract obtained on the same material, with an optimized process involving microwave (1000 W) and ultrasound (200 W), with a temperature plateau between 116°C and 121°C, presented a phenol content of 130 ppm (mg oleuropein equivalent/kg) and a antiradical activity of 331 µmoles Trolox equivalent/kg.

### Example 3

125 g of powder of olive leaves, with a water content of 20% (m/m), were poured in a glass flask.

Step a0) was carried out during 11 minutes, under Near InfraRed with a power of 1000 W, with a regular manual agitation. The temperature reached was 88°C.

The plant material was then mixed to 500 g of deodorized sunflower oil, at a ratio 1:4.

An agitated diffusion was performed in the closed glass flask, under a nitrogen sweep and an agitation of 200 rpm, during 30 minutes.

The oily extract was separated from the exhausted plant material by spin-drying, at 1800 rpm for 10 minutes on a filter clothe of 5 µm porosity, and then on a cellulosic paper of 1 µm porosity.

The total phenolic content was 125 ppm (mg oleuropein equivalent/kg) for the oily extract obtained with the process.

### Example 4

20 g of powder of olive leaves, with a water content of 20% (m/m), were mixed with 80 g of deodorized sunflower oil, at a ratio 1:4. The mix was poured in an open beaker, with a diving probe conducing the low frequency vibration of an InfraSonic device set up on the top of the lid.

Step b) was carried out during 15 minutes, in the presence of InfraSound with a power of 9.5 W and frequencies of 50 Hz and 100 Hz, with no agitation. No temperature elevation occurred.

The total phenolic content was 118 ppm (mg oleuropein equivalent/kg) for the extract obtained at the frequency of 50 Hz and 167 ppm (mg oleuropein equivalent/kg), at the frequency of 100 Hz.

When step b) was performed during 90 minutes, the phenol content of the extract was 216 ppm (mg oleuropein equivalent/kg) and the antiradical activity of the oily extract was 460 µmoles Trolox equivalent/kg.

The microbial activity of the extract was compliant with the norms.

### Example 5

The extraction mix was prepared as in Example 2.

Step b) was carried out during 21 minutes, under Near InfraRed with a power of 1000 W, with a mechanical agitation of 200 rpm. The temperature reached was 163°C.

The InfraRed was stopped, and the Erlenmeyer flask was set on a vibrating table, emitting low frequency agitation from the walls of the flask.

A second step b) was carried out for 90 minutes, during the return to room temperature, with InfraSound at a power of 430 W and a frequency of 50 Hz.

The total phenolic content of the extract was 771 ppm (mg oleuropein equivalent/kg).

The peroxide value was of 7.2 +/- 0.5 meqO₂/kg, with nitrogen sweep.

The antiradical activity of the oily extract was 2506 µmoles Trolox equivalent/kg, which is higher than the addition of the antiradical activity of the IRAE extract and the ISAE extract performed separately in the same conditions, of respectively 1652 µmoles Trolox equivalent/kg (see Example 1) and 460 µmoles Trolox equivalent/kg (see Example 4).

When sonic vibration by InfraSound was performed before excitation by InfraRed, the total phenolic content was 669 ppm (mg oleuropein equivalent/kg) for the oily extract and the peroxide value was of 8.5 +/- 0.5 meqO₂/kg, with nitrogen sweep.

### Example 6

The same process as Example 5 was performed on 155 g of carrot roots, prepared as in Example 1, mixed with 465 g of virgin soy oil, at a ratio 1:3.

Step b) was carried out during 15 minutes, under Near InfraRed with a power of 1000 W and InfraSound at a power of 9.5 W and a frequency of 100 Hz. The temperature reached was 81°C. Carotenoids content was 119 ppm (mg β-carotene equivalent/kg).

An oily extract obtained on the same material, with an optimized process involving microwave (1000 W) and ultrasound (200 W), with a temperature plateau between 108°C and 120°C, presented the same carotenoids content.

### Example 7

300 g of propolis, with a water content of 0.85% (m/m), comminuted in a powdery form with a knife mill, were mixed with 300 g of deodorized olive oil, at ratio 1:1.

The mix was poured to a silicone recipient with open head, no nitrogen sweep and no agitation. A tungsten-halogen quartz tube was placed on the top of the recipient.

Step b) was carried out during 20 minutes, under Near InfraRed with a power of 800 W. The temperature reached was 106°C. The liquid phase was separated from the exhausted raw material by filtration on 6 head caps.

The obtained extract was semi-solid at room temperature and its total phenolic content was 26 256 ppm (mg oleuropein equivalent/kg).

### Example 8

16.7 g of pomegranate leaves with a water content of 20% (m/m), cut with a hammer mill with a sieve of 800 µm, were mixed with 83.5 g of a solvent composed by 71.0 g of virgin jojoba liquid wax and 12.5 g of polyglyceryl-3-diisostearate, at a ratio 1:5.

A ceramic InfraRed lightbulb containing a metallic alloy resistance was placed at the side of the beaker containing the extracting mix, and an InfraSonic device with a diving probe was placed at the top of the beaker.

Step b) was carried out for 37 minutes, under Far InfraRed at a power of 150 W with InfraSound at a power of 9.5 W and a frequency of 100 Hz. The temperature went from 21°C to 52°C.

The content in ellagic acid, determined by HPLC, was 22 ppm (mg ellagic acid equivalent/kg).

The same protocol was carried out with a hardened glass InfraRed lightbulb containing a metallic alloy resistance.

Step b) was carried out for 30 minutes, under Far InfraRed at a power of 150 W with InfraSound at a power of 9.5 W and a frequency of 100 Hz. The temperature went from 21°C to 81°C.

The content in ellagic acid, determined by HPLC, was 53 ppm (mg ellagic acid equivalent/kg).

## Claims

1. A process of extraction of natural compounds from raw materials having a water content between 0 % and 20 %, said process comprising:
a) mixing and impregnating the raw materials by dispersing said raw materials with a liquid extracting solvent, under agitation, the ratio between the raw materials and the solvent being comprised between 1:1 and 1:20 in mass/mass,
wherein step a) is preceded by:
a0) activating by InfraRed the raw materials, said raw materials being in the form of a powder, at a temperature below their ignition point, with an agitation; and/or
wherein step a) is followed by:
b) extracting the natural compounds by submitting the mix obtained at step a) to excitation by InfraRed and/or sonic vibration by InfraSound, under an agitation.

2. The process according to claim 1, wherein step b) can be repeated more than once.

3. The process according to claim 2, wherein when step b) is repeated more than once, a passive diffusion step is performed between them at room temperature or with the cooling of the extracting tank.

4. The process according to any of the preceding claims wherein:
- step a0) is performed for a duration of between 5 and 30 minutes; and/or
- step b) is performed for a duration of between 5 and 90 minutes.

5. The process according to any of the preceding claims, wherein step b) comprises extracting the natural compounds by submitting the mix obtained at step a) to excitation by InfraRed together with heating by InfraRed convection between 20°C and 160°C.

6. The process according to any of the preceding claims, wherein when performed together, InfraRed and InfraSound are performed sequentially or simultaneously.

7. The process according to any of the preceding claims, wherein the power of InfraRed is from 50 W to 2000 W by kilogram of mix.

8. The process according to any of the preceding claims, wherein the wavelength of InfraRed is from near InfraRed to far InfraRed, i.e. from 0.76 µm to 1000 µm and/or wherein the source of InfraRed is a filament made of tungsten, carbon or iron alloys, embedded in a quartz tube filled with halogen gas, a chromium alloy filament inside a metal or quartz sheath, or a metallic alloy resistance embedded in a hardened glass or a ceramic element (plate or bulb).

9. The process according to any of the preceding claims, wherein the power of InfraSound is from 10 W to 1000 W by kilogram of mix.

10. The process according to any of the preceding claims, wherein the frequency of InfraSound is between 0.1 Hz and 100 Hz.

11. The process according to any of the preceding claims, wherein the source of InfraSound are transducers chosen from piezoelectric and inverse piezoelectric effect devices, magnetostrictive devices, sliding magnetic/moving coils or electrostatic devices.

12. The process according to any of the preceding claims, wherein said raw materials are all or a part of a plant, a mineral, a fungus, an alga, a bacterium or of animal origin, an industrial byproduct that has not been subjected to chemical treatment or a transformation modifying its chemical composition, or a combination of them.

13. The process according to any of the preceding claims, wherein said solvent is chosen from oily solvents, such as vegetable liquid oil, vegetable butter, vegetable fat, natural wax, fatty acid, fatty ester, fatty alcohol, glyceride, any other fatty acid derivative or a combination of them.

14. The process according to any of the preceding claims, wherein said natural compounds are chosen from phenolic compounds such as monophenols, polyphenols, flavonoids, phenolic acids, catechin and epicatechin, flavones and flavonols; diterpenes; vitamins; tannins; waxes; fatty acids; essential oils; organic acids; hydrophobic proteins; pigments such as carotenoids, xanthophyll, lutein, zeaxanthin and chlorophyll; unsaponifiable compounds such as free or esterified phytosterols, fatty alcohols, triterpenes and squalene; polar lipids such as phospholipids, glycolipids and sphingolipids; enzymes; co-enzymes; oligo-elements; minerals; organic salts and a mix of one or more of these compounds.

15. A pharmaceutical, cosmetic, cosmeceutical, nutraceutical or food composition, comprising an extract enriched in natural compounds obtained by the process according to any of the preceding claims.
